# EUROPÄISCHE PATENTSCHRIFT

(11) **EP 2 026 868 B1**
(45) Veröffentlichungstag und Bekanntmachung des Hinweises auf die Patenterteilung: **23.02.2011**
(21) Anmeldenummer: 07726030.5
(22) Anmeldetag: 14.06.2007
(51) Int. Cl.: A61M 37/00

(54) **IMPLANTIERBARE VORRICHTUNG**
IMPLANTABLE DEVICE
DISPOSITIF IMPLANTABLE

(30) Priorität: 14.06.2006 DE 202006009375 U
(43) Veröffentlichungstag der Anmeldung: 25.02.2009
(73) Patentinhaber: Reinmüller, Johannes, 65193 Wiesbaden (DE)
(72) Erfinder: Reinmüller, Johannes, 65193 Wiesbaden (DE)
(74) Vertreter: Weiss, Wolfgang
(86) Internationale Anmeldenummer: PCT/EP2007/005268
(87) Internationale Veröffentlichungsnummer: WO 2007/144181

(56) Entgegenhaltungen:
- WO-A-00/59571
- DE-A1-102004 027 461
- DE-C1- 10 139 493
- US-A1- 2004 101 479
- US-A1- 2005 165 347
- DAVID MARGOLICK: "At the Bar; For lawyers and judges in the fight against drugs, the repugnant becomes run of the mill." THE NEW-YORK TIMES, [Online] 24. Juli 1992 (1992-07-24), XP002448971 Gefunden im Internet: URL:HTTP://QUERY.NYTIMES.COM/GST/FULLPAGE. HTML?SEC=HEALTH&RES=9E0CEED81E31F937A15754 C0A964958260> [gefunden am 2007-08-30]

## Beschreibung

Die vorliegende Erfindung betrifft eine implantierbare Vorrichtung für Wertobjekte.

Diamanten und andere Edelsteine werden heute oftmals in Form von so genannten Piercings an geeigneten Körperteilen oberflächlich als Schmuck angebracht. Die dabei entstehenden Verletzungen heilen regelmäßig gut ab.

In Romanen und Spielfilmen gibt es weiterhin Beschreibungen, worin sich Figuren der Handlung Wertobjekte unter die Haut einpflanzen lassen, um diese zu verbergen und ständig für Notsituationen zur Verfügung zu haben. Rein praktisch können solche Wertobjekte durch einen chirurgischen Eingriff implantiert werden, wenn dabei die Regeln für die Implantation von Fremdkörpern, z.B. die Sterilität des Implantats, beachtet werden. Ein Problem nach der Implantation von Diamanten oder anderen Edelsteinen im Weichgewebe entsteht jedoch durch die Neigung dieser Fremdkörper zur Wanderung. Dadurch kann es zur Abstoßung des Fremdkörpers nach außen oder zum Eindringen in tiefere Gewebeschichten mit Verletzungen wichtiger anatomischer Strukturen kommen.

Gemäß vorliegender Erfindung wird die Implantation von Wertobjekten dadurch gelöst, dass sie in ein geeignetes Hüllmaterial eingeschlossen werden, welches ausreichende Biokompatibilität, Beständigkeit und Stabilität am lmplantationsort gewährleistet. Ferner ist die implantierbare Vorrichtung derart ausgestaltet, dass sie eine einfache Applikation, Identifizierbarkeit und leichte Entfernbarkeit gewährleistet.

Ein Gegenstand der Erfindung ist daher eine implantierbare Vorrichtung, die ein oder mehrere Wertobjekte in einem Behältnis mit einer äußeren schlauchförmigen Hülle mit einer oder mehreren Verdickungen aus einem biokompatiblen Kunststoffmaterial enthält, wobei das Kunststoffmaterial einen Silikonkunststoff und/oder ein Verbundmaterial auf Basis eines Silikonkunststoffs umfasst. Die implantierbare Vorrichtung ist sowohl beim Menschen als auch bei Tieren anwendbar.

Wertobjekte im Sinne der vorliegenden Erfindung können beispielsweise Edelsteine, insbesondere Diamanten, und/oder Informationsträger, z.B. Speicherchips, Mikrodokumente etc. sein. Falls mehrere Wertobjekte in Inneren der Vorrichtung angeordnet sind, sollten sie sich nicht berühren. Die Abmessungen der implantierbaren Vorrichtung sind vorzugsweise in der Länge von mindestens 20 mm, vorzugsweise von 30 mm, vorzugsweise 50 mm, vorzugsweise 100 mm, vorzugsweise 150 mm, bis etwa 250 mm, vorzugsweise bis 200 mm. Der Durchmesser der Vorrichtung sollte sich im Bereich von etwa 2 mm bis etwa 30 mm bewegen, vorzugsweise 4 bis 20 mm. Er kann auch kleiner sein, z.B. im Bereich von 4 bis 15 mm oder 4 bis 10 mm.

Die implantierbare Vorrichtung enthält eine äußere schlauchförmige Hülle aus einem biokompatiblen Kunststoffmaterial. Bevorzugt umfasst das Kunststoffmaterial einen Silikonkunststoff, z.B. Polydimethylsilikon, oder verwandte Kunststoffe, z.B. halogenierte und/oder mit aromatischen Gruppen substituierte Silikonkunststoffe. Auch alle anderen biokompatiblen Kunststoffmaterialien, wie etwa Polyethylene, Polypropylene, Polyurethane, Polyamide, z.B. Polyacrylamide wie etwa PMMA oder Nylon, Polyterephthalate, fluorierte Kunststoffe, wie etwa Polytetrafluorethylen oder Copolymere davon, sind geeignet.

Zur Verbesserung der mechanischen Belastbarkeit kann das Hüllmaterial auch ein Verbundmaterial sein, bei dem das Kunststoffmaterial, z.B. der Silikonkunststoff, mit Fasern, wie etwa Textilfasern, metallischen Fasern, Kunststofffasern und/oder Carbonfasern, verstärkt ist.

Die Hülle ist vorzugsweise aus einem flexiblen Kunststoffmaterial ausgebildet.

Statt einer homogenen äußeren Hülle, z.B. aus Silikonkautschuk, können auch Gewebe verwendet werden, z.B. PTFE-Gewebe wie sie für Gefäßprothesen bekannt sind, oder offenmaschige Gewebe aus sich überkreuzenden Kunststoff- oder Metallfäden in Form von Haltenetzen.

Die Hülle hat vorzugsweise eine Dicke von etwa 0,2 mm bis etwa 4,0 mm, vorzugsweise von 0,5 mm, vorzugsweise 1,0 mm, vorzugsweise 2,0 mm bis 2,5 mm, vorzugsweise bis 3 mm, vorzugsweise bis 3,5 mm. Andererseits kann die Hülle auch das Innere der Vorrichtung ausfüllen, so dass die Wertobjekte vollständig in die Hülle eingebettet sind.

Die innere und/oder äußere Oberfläche der Kunststoffhülle ist vorzugsweise glatt. Sie kann aber auch aufgeraut, gelförmig oder schaumförmig sein. Besonders bevorzugt ist zumindest die äußere Oberfläche der Hülle glatt. Die Oberfläche, insbesondere die äußere Oberfläche, kann zur Verbesserung der Biokompatibilität und der Gleitfähigkeit chemisch oberflächenmodifiziert, z.B. hydrophilisiert, sein. Geeignete hydrophile Gruppen sind etwa Hydroxygruppen und/oder Polyethylenoxidgruppen. Ferner kann die Oberfläche der Hülle, insbesondere die äußere Oberfläche, zumindest teilweise mit einer biologischen Substanz beschichtet sein, welche die Biokompatibilität verbessert. Als biologische Substanzen kommen beilspielsweise Gtykosaminogtykane, wie Heparin und/oder Hyaluronsäure, infrage. Die biologischen Substanzen können adsorptiv, durch ionische Bindungen und/oder durch kovalente Bindungen auf der Oberfläche der Hülle immobilisiert sein.

Die die Wertobjekte enthaltende schlauchförmige Hülle kann grundsätzlich einen beliebigen Querschnitt, beispielsweise einen runden oder abgeplatteten, z.B. ovalen, kreuzförmigen oder sternförmigen Querschnitt, aufweisen. Besonders bevorzugt ist ein runder, insbesondere ein drehrunder Querschnitt.

Zusätzlich zu der die Wertobjekte enthaltenden Hülle kann die implantierbare Vorrichtung noch an zumindest einem Ende einen fadenförmigen Fortsatz aufweisen, der zur Lagemarkierung innerhalb des Körpers dient. Ein solcher fadenförmiger Fortsatz kann beispielsweise aus Verstärkungsfasern gebildet werden, die in das Kunststoffmaterial der Hülle eingearbeitet sind.

In das Innere einer solchen Hülle können die Wertobjekte einzeln oder in Serie eingebracht werden. Sofern die Oberflächen der Wertobjekte glatt und kantenfrei sind (Kabochonform, Rohdiamant), können sie in die schlauchförmige Hülle unter elastische Spannung eingesetzt werden, d.h. der Durchmesser der elastischen Hülle ist kleiner als der Durchmesser der rundlichen oder ovalären Wertobjekte. Damit werden die Wertobjekte durch die elastische Spannung der Hülle ortsstabil fixiert. Es resultiert ein schotenförmiges lmplantat, welches nach Einwachsen im Weichgewebe durch eine Bindegewebshülle anatomisch ortsstabil bleibt.

Wertobjekte wie geschliffene Diamanten und Edelsteine oder Elektronikbauteile, die keine kantenfreie Oberflächen besitzen, sollten vor dem Einbringen in die Hülle in einen kugelförmigen oder ovalären Zustand überführt werden. Hierzu werden sie entweder in Formkörper, wie etwa Schäume, Gele oder Festmassen aus Kunststoff, eingehüllt oder sie werden in passgenaue Kapseln eingesetzt, die durch Schraubverschluss oder durch Kleben geschlossen werden. Die Formkörper können jeweils ein oder mehrere Wertobjekte enthalten. Wenn mehrere Wertobjekte in einem einzigen Formkörper vorliegen, sollte ein unmittelbarer Kontakt durch ein Trennmaterial, z.B. eine Folie, verhindert werden. Die Formkörper mit den Wertobjekten können sodann in die Außenhüllen eingesetzt werden, so dass wieder die erwünschte Schotenform entsteht. Die Schotenform ist bevorzugt, aber nicht Bedingung.

Denkbar ist auch eine Ausführungsform, bei der eine Hülle, z.B. ein Silikonelastomerschlauch, mit einem hochkohäsiven Silikongel befüllt wird, in welchem die Wertgegenstände (Brillanten) auf Abstand eingebettet sind. Denkbar sind auch Befüllungen bereits gebräuchlicher Silikonimplantate (Mamma-Implantat) mit Wertobjekten im Silikongelbett.

Zusätzlich zu den Wertobjekten kann die implantierbare Vorrichtung einen Identifikator enthalten, z.B. einen Transponder, der in Verbindung mit einem geeigneten Signalgeber eine Identifizierung und/oder Lokalisierung des Implantats ermöglicht.

Das Schutzrecht bezieht sich auch auf eine Vorrichtung zum Implantieren des Wertobjektbehälters. Dies ist vorzugsweise eine glattwandige Kanüle aus Metall oder Kunststoff, die den Wertobjektbehälter aufnehmen kann, vorzugsweise passgenau, d.h. zwischen Innenwand der Kanüle und der Außenwand des Wertobjektbehälters verbleibt ein kapillarer Spalt. Ein Ende der Kanüle ist offen, rechtwinklig oder abgeschrägt, nicht in jedem Falle aber bevorzugt, stumpf mit gebrochenen Kanten, ausnahmsweise auch scharf und geschliffen. Das andere Ende ist konisch verjüngt und bevorzugt mit einem Luer-Konus-Aufsatz ausgestattet.

Die Implantation des Wertobjektbehälters geschieht wie folgt :
- Örtliche Betäubung (ausnahmsweise Narkose),
- Stichinzision in der Haut,
- Einführen der mit dem Wertobjektbehälter gefüllten Kanüle in das Unterhautfettgewebe oder die Muskulatur mit dem offenen Ende,
- Aufsetzen einer handelsüblichen Spritze mit physiologischer Kochsalzlösung auf den Luer-Konus, durch Druck auf den Spritzenkolben treibt die Kochsalzlösung das Wertobjektimplantat hydraulisch aus in das Gewebe, gleichzeitig wird die Kanüle zurückgezogen.
- Anstelle der Kochsalzspritze zum Austreiben des Implantats aus dem Applikator kommt auch ein passgenauer Stempel aus Kunststoff oder Metall infrage. In diesem Fall ist der Applikator vorzugsweise an beiden Enden offen, der Luer-Ansatz fehlt.
- Die Wunde in der Haut wird durch Naht oder mit Kleber verschlossen.

Weiterhin soll die Erfindung durch die folgenden Figuren erläutert werden.

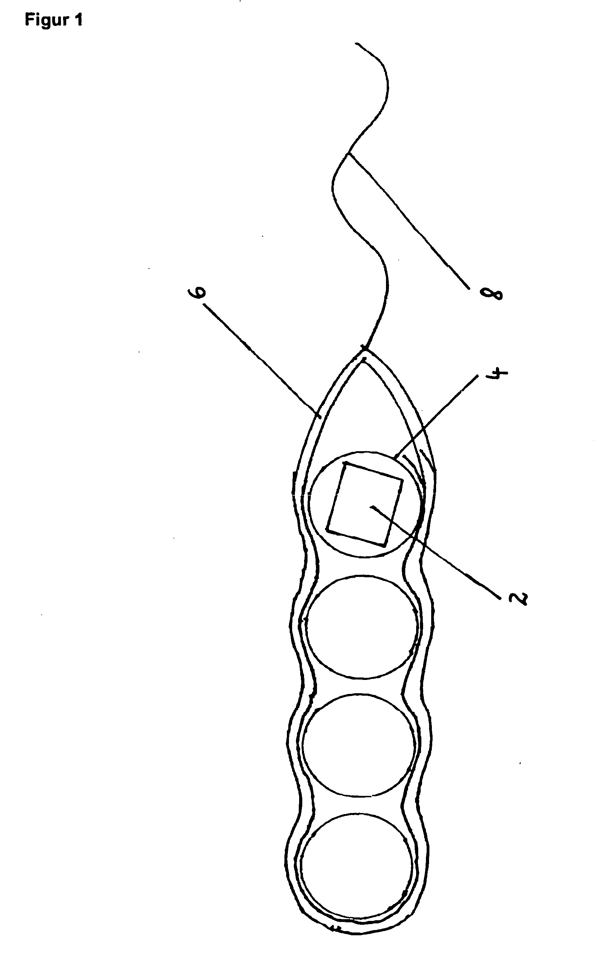

**Figur 1** zeigt eine bevorzugte Ausführungsform der implantierbaren Vorrichtung. Die Wertobjekte (2), z.B. Speicherchips, sind in einem Formkörper (4), z.B. einer Kapsel aus Polyethylen, angeordnet. Um die Formkörper (4) ist eine äußere Hülle (6), z.B. aus einem Silikonkunststoff, so angeordnet, dass die schlauchförmige Vorrichtung mehrere Verdickungen aufweist und somit eine Schotenform besitzt. An einem Ende der Vorrichtung ist ein Faden (8) angeordnet.

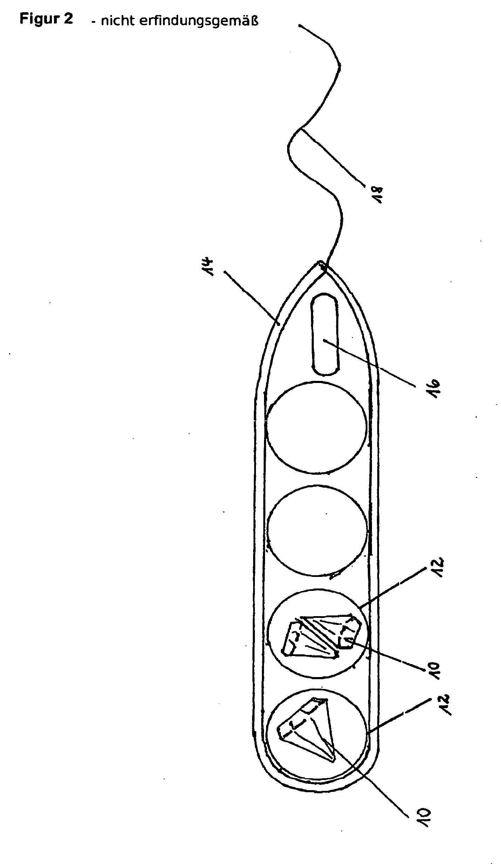

**Figur 2** zeigt eine nicht erfindungsgemäße Ausführungsform der implantierbaren Vorrichtung. Die Wertobjekte (10), z.B. Brillanten, sind in Formkörpern (12) eingeschlossen. Die Innenbehältnisse befinden sich in einer schlauchförmigen äußeren Hülle (14), z.B. aus Silikonkautschuk. In dieser Ausführungsform ist die Vorrichtung ohne Verdickungen ausgebildet, so dass sie eine Zäpfchenform aufweist. Ferner ist in der Hülle (14) ein Identifikator (16), z.B. ein RFID-Chip, angeordnet. Weiterhin weist die Vorrichtung einen Faden (18) auf.

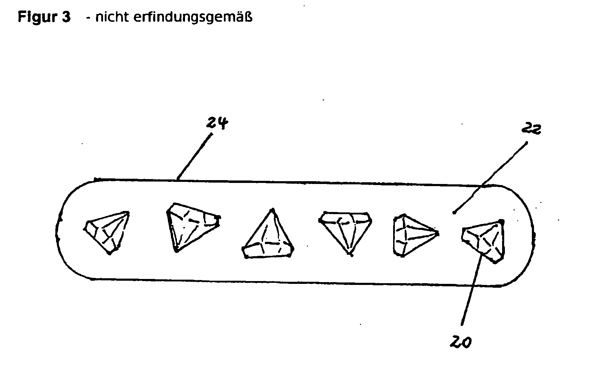

**Figur 3** zeigt noch eine nicht erfindungsgemäße Ausführungsform der implantierbaren Vorrichtung. Mehrere Wertobjekte (20), z.B. Brillanten, sind in einer Kunststoffmasse (22), z.B. aus Silikonkunststoff, eingebettet, die von einer Hülle umgeben sein kann oder selbst die Hülle für die Wertobjekte darstellt. Die Vorrichtung weist eine glatte Form ohne Verdickungen auf.

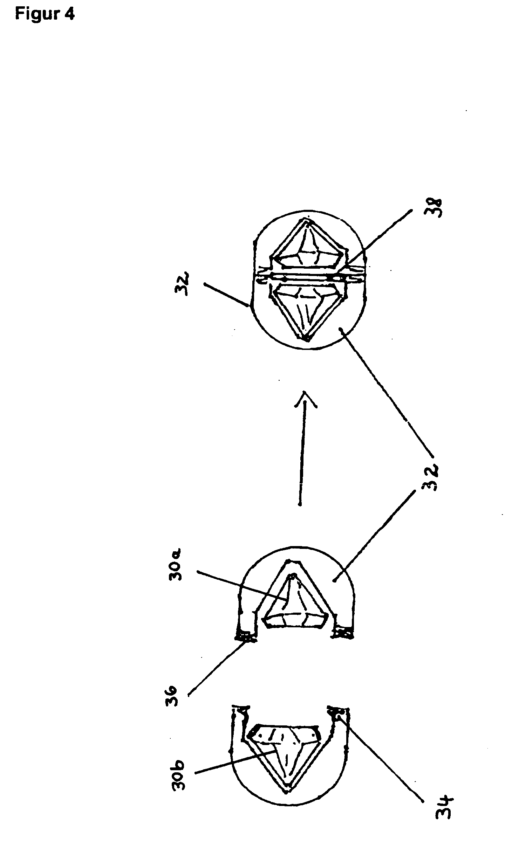

**Figur 4** zeigt eine bevorzugte Ausführungsform für einen Formkörper zur Einbettung von Wertobjekten (30 a, b), wie etwa Brillanten. Der Formkörper ist in Form einer Kapsel (32), z.B. aus einem Acrylkunststoff wie PMMA, oder Carbon, ausgebildet und kann ein Außengewinde (34) und ein dazu passendes Innengewinde (36) aufweisen. Nach Befüllen der Kapsel mit den Wertobjekten und gegebenenfalls Einfügen eines Abstandhalters (38), z.B. einer Silikonfolie, wird die Kapsel zusammengeschraubt und in eine Hülle (nicht gezeigt) eingesetzt.

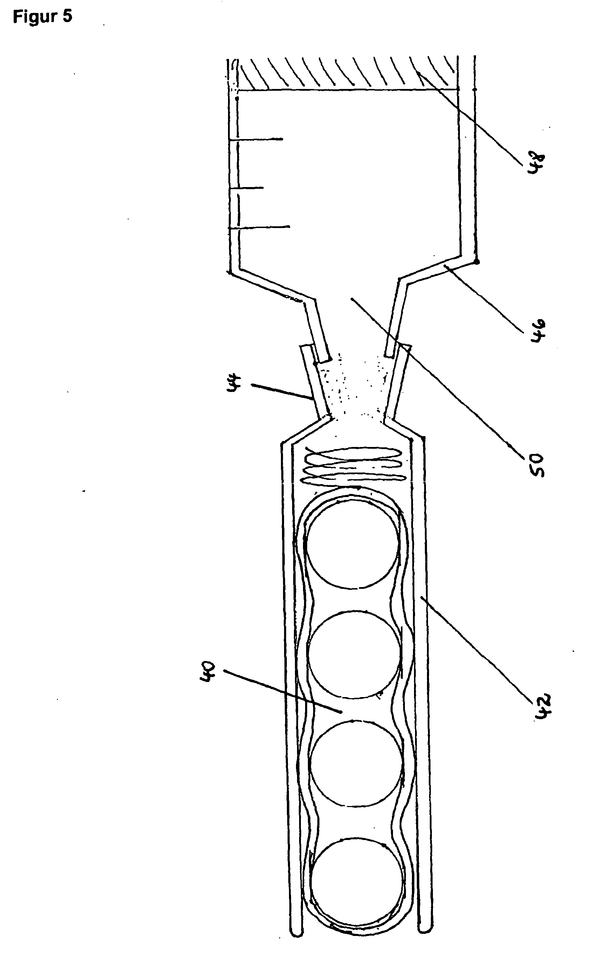

**Figur 5** zeigt einen Applikator, bei dem die implantierbare Vorrichtung (40) in einer Kanüle (42) mit einem Luer-Konus-Aufsatz (44) aufgenommen ist. Die Kanüle (42) wird auf eine Spritze (46) mit einem Kolben (48) aufgesetzt. In der Spritze ist eine pharmazeutisch verträgliche Flüssigkeit, z.B. eine physiologische Kochsalzlösung (50), enthalten. Durch Druck auf den Kolben (46) treibt die Flüssigkeit die implantierbare Vorrichtung (40) in das Gewebe.

## Patentansprüche

1. Implantierbare Vorrichtung,
**dadurch gekennzeichnet,**
**dass** sie ein oder mehrere Wertobjekte in einem Behältnis mit einer äußeren schlauchförmigen Hülle mit einer oder mehreren Verdickungen aus einem biokompatiblen Kunststoffmaterial enthält, wobei das Kunststoffmaterial einen Silikonkunststoff und/oder ein Verbundmaterial auf Basis eines Silikonkunststoffs umfasst. :

2. Vorrichtung nach Anspruch 1,
**dadurch gekennzeichnet,**
**dass** die Wertobjekte Edelsteine, insbesondere Diamanten, umfassen.

3. Vorrichtung nach Anspruch 1 oder 2,
**dadurch gekennzeichnet,**
**dass** die Wertobjekte Informationsträger umfassen.

4. Vorrichtung nach einem der Ansprüche 1 bis 3,
**dadurch gekennzeichnet,**
**dass** die Hülle einen runden, ovalen, kreuzförmigen oder sternförmigen Querschnitt aufweist.

5. Vorrichtung nach einem der Ansprüche 1 bis 4,
**dadurch gekennzeichnet,**
**dass** die Oberfläche, insbesondere zumindest die äußere Oberfläche der Hülle, glatt ist.

6. Vorrichtung nach einem der Ansprüche 1 bis 5,
**dadurch gekennzeichnet,**
**dass** die Oberfläche, insbesondere die äußere Oberfläche der Hülle, zumindest teilweise chemisch modifiziert ist.

7. Vorrichtung nach einem der Ansprüche 1 bis 6,
**dadurch gekennzeichnet,**
**dass** die Oberfläche, insbesondere die äußere Oberfläche der Hülle, zumindest teilweise mit einer biologischen Substanz, insbesondere mit einem Glykosaminoglykan wie Heparin und/oder Hyaluronsäure, beschichtet ist.

8. Vorrichtung nach einem der Ansprüche 1 bis 7,
**dadurch gekennzeichnet,**
**dass** sie an zumindest einem Ende einen fadenförmigen Fortsatz aufweist.

9. Vorrichtung nach einem der Ansprüche 1 bis 8,
**dadurch gekennzeichnet,**
**dass** sie ein einziges Wertobjekt enthält.

10. Vorrichtung nach einem der Ansprüche 1 bis 8,
**dadurch gekennzeichnet,**
**dass** sie mehrere Wertobjekte in Serie angeordnet enthält.

11. Vorrichtung nach Anspruch 10,
**dadurch gekennzeichnet,**
**dass** die Wertobjekte innerhalb der Hülle derart angeordnet sind, dass sie nicht miteinander in Kontakt stehen.

12. Vorrichtung nach einem der Ansprüche 1 bis 11,
**dadurch gekennzeichnet,**
**dass** die Wertobjekte innerhalb der Hülle in Formkörper eingebettet sind.

13. Vorrichtung nach Anspruch 12,
**dadurch gekennzeichnet,**
**dass** die Formkörper Schäume, Gele, Festmassen oder Kapseln umfassen.

14. Vorrichtung nach einem der Ansprüche 1 bis 13,
**dadurch gekennzeichnet,**
**dass** sie weiterhin einen Identifikator umfasst.

15. Vorrichtung nach einem der Ansprüche 1 bis 14,
**dadurch gekennzeichnet,**
**dass** sie in einer Implantierkanüle angeordnet ist.

16. Vorrichtung nach Anspruch 15,
**dadurch gekennzeichnet,**
**dass** sie passgenau von der Implantierkanüle aufgenommen ist.

## Claims

1. An implantable device, **characterised in that** it contains one or more valuable objects in a container with an outer tubular envelope with one or more thickened portions made from a biocompatible plastics material, the plastics material comprising a silicone plastic and/or a composite material based on a silicone plastic.

2. A device according to claim 1, **characterised in that** the valuable objects comprise precious stones, in particular diamonds.

3. A device according to claim 1 or claim 2, **characterised in that** the valuable objects comprise information storage media.

4. A device according to any one of claims 1 to 3, **characterised in that** the envelope has a round, oval, cruciform or stellate cross-section.

5. A device according to any one of claims 1 to 4, **characterised in that** the surface, in particular at least the outer surface of the envelope, is smooth.

6. A device according to any one of claims 1 to 5, **characterised in that** the surface, in particular the outer surface of the envelope, is at least in part chemically modified.

7. A device according to any one of claims 1 to 6, **characterised in that** the surface, in particular the outer surface of the envelope, is coated at least in part with a biological substance, in particular with a glycosaminoglycan such as heparin and/or hyaluronic acid.

8. A device according to any one of claims 1 to 7, **characterised in that** it comprises a thread-like extension at at least one end.

9. A device according to any one of claims 1 to 8, **characterised in that** it contains a single valuable object.

10. A device according to any one of claims 1 to 8, **characterised in that** it contain a plurality of valuable objects arranged in series.

11. A device according to claim 10, **characterised in that** the valuable objects are arranged within the envelope in such a manner that they are not in contact with one another.

12. A device according to any one of claims 1 to 11, **characterised in that** the valuable objects are embedded in mouldings within the envelope.

13. A device according to claim 12, **characterised in that** the mouldings comprise foams, gels, solid compositions or capsules.

14. A device according to any one of claims 1 to 13, **characterised in that** it furthermore comprises an identifier.

15. A device according to any one of claims 1 to 14, **characterised in that** it is arranged in an implantation cannula.

16. A device according to claim 15, **characterised in that** it is accommodated by the implantation cannula with a perfect fit.

## Revendications

1. Dispositif implantable,
**caractérisé en ce**
**qu'**il contient un ou plusieurs objets de valeur dans un récipient doté d'une enveloppe extérieure tubulaire avec un ou plusieurs épaississements en un matériau plastique biocompatible, le matériau plastique comportant un plastique silicone et/ou un matériau composite à base d'un plastique silicone.

2. Dispositif selon la revendication 1,
**caractérisé en ce**
**que** les objets de valeur sont des pierres précieuses, en particulier des diamants.

3. Dispositif selon la revendication 1 ou 2,
**caractérisé en ce**
**que** les objets de valeur comportent des supports d'informations.

4. Dispositif selon l'une quelconque des revendications 1 à 3,
**caractérisé en ce**
**que** l'enveloppe présente une section transversale ronde, ovale, en croix ou en étoile.

5. Dispositif selon l'une quelconque des revendications 1 à 4,
**caractérisé en ce**
**que** la surface, en particulier au moins la surface extérieure de l'enveloppe est lisse.

6. Dispositif selon l'une quelconque des revendications 1 à 5, **caractérisé en ce que** la surface, en particulier la surface extérieure de l'enveloppe est modifiée au moins en partie chimiquement.

7. Dispositif selon l'une quelconque des revendications 1 à 6,
**caractérisé en ce**
**que** la surface, en particulier la surface extérieure de l'enveloppe, est revêtue au moins en partie d'une substance biologique, en particulier d'un glycosaminoglycane tel que l'héparine et/ou l'acide hyaluronique.

8. Dispositif selon l'une quelconque des revendications 1 à 7,
**caractérisé en ce**
**qu'**il présente un prolongement filiforme au niveau d'au moins une extrémité.

9. Dispositif selon l'une quelconque des revendications 1 à 8,
**caractérisé en ce**
**qu'**il contient un objet de valeur unique.

10. Dispositif selon l'une quelconque des revendications 1 à 8,
**caractérisé en ce**
**qu'**il contient plusieurs objets de valeur disposés en série.

11. Dispositif selon la revendication 10,
**caractérisé en ce**
**que** les objets de valeur sont disposés dans l'enveloppe de telle manière qu'ils n'entrent pas en contact les uns avec les autres.

12. Dispositif selon l'une quelconque des revendications 1 à 11,
**caractérisé en ce**
**que** les objets de valeur dans l'enveloppe sont insérés dans des corps moulés.

13. Dispositif selon la revendication 12,
**caractérisé en ce**
**que** les corps moulés comportent des mousses, des gels, des masses solides ou des capsules.

14. Dispositif selon l'une quelconque des revendications 1 à 13,
**caractérisé en**
**ce qu'**il comporte en outre un identificateur.

15. Dispositif selon l'une quelconque des revendications 1 à 14,
**caractérisé en ce**
**qu'**il est disposé dans une canule implantable.

16. Dispositif selon la revendication 15,
**caractérisé en ce**
**qu'**il est reçu en ajustage précis par la canule implantable.
